(19) Europäisches Patentamt
European Patent Office
Office européen des brevets

(11) **EP 4 023 266 B1**

(12) **EUROPEAN PATENT SPECIFICATION**

(45) Date of publication and mention
of the grant of the patent:
**03.07.2024  Bulletin 2024/27**

(21) Application number: **21204532.2**

(22) Date of filing: **25.10.2021**

(51) International Patent Classification (IPC):
***A61L 27/26*** *(2006.01)*      ***A61L 27/38*** *(2006.01)*
***A61L 27/58*** *(2006.01)*

(52) Cooperative Patent Classification (CPC):
(C-Sets available)
**A61L 27/38; A61L 27/26; A61L 27/3895;**
**A61L 27/58**                                      (Cont.)

(54) **NON-FIBROUS FILM AND CELL SHEET**

NICHTFASERIGER FILM UND ZELLFOLIE

FILM NON FIBREUX ET FEUILLE DE CELLULE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.12.2020  TW 109147086**

(43) Date of publication of application:
**06.07.2022  Bulletin 2022/27**

(73) Proprietor: **Industrial Technology Research
Institute
Chutung, Hsinchu 310401 (TW)**

(72) Inventors:
  • **Liou, Yu-Bing**
    **Hsinchu City (TW)**
  • **Liao, Chih-Ching**
    **Hsinchu County (TW)**
  • **Hsu, Hsin-Yi**
    **Taoyuan City (TW)**
  • **Shen, Ying-Wen**
    **Miaoli County (TW)**
  • **Teng, Yun-Chung**
    **Kaohsiung City (TW)**
  • **Shen, Hsin-Hsin**
    **Hsinchu County (TW)**
  • **Chen, Yi-Chen**
    **Hsinchu City (TW)**

(74) Representative: **Michalski Hüttermann & Partner
Patentanwälte mbB
Kaistraße 16A
40221 Düsseldorf (DE)**

(56) References cited:
  EP-A1- 3 366 700      CN-A- 107 325 307
  CN-A- 108 042 856     CN-A- 109 125 808
  US-A1- 2017 266 345

(52) Cooperative Patent Classification (CPC): (Cont.)

C-Sets
**A61L 27/26, C08L 67/04;**
**A61L 27/26, C08L 89/06**

## Description

### TECHNICAL FIELD

The present disclosure relates to a non-fibrous film and a cell sheet.

### BACKGROUND

The primary purpose of regenerative medicine cell therapy is to replace, repair, improve or regenerate various tissues and organs of the body. In traditional regenerative medicine, a cell suspension or its combination with a cell complex carrier scaffold is usually used to deliver cells to the affected area. Although traditional regenerative cell therapy have greatly promoted the development of regenerative medicine, the harvest of cell suspensions often requires trypsin to suspend the cells, and this step usually damaged the surface proteins of the cells. Once the surface proteins of the cells are damaged, cell function is affected. When cell surface proteins are disrupted, the intercellular and extracellular matrix (ECM) interactions are reduced. These intercellular interactions and the production of extracellular matrix are essential to maintain tissue function. The use of trypsin to harvest cell suspensions will have a negative effect on the adhesion and proliferation of cells, resulting in uneven distribution of cells at the implantation site or reduced repair efficiency after injection of cell suspensions, and even causing adverse reactions such as inflammation at the implantation site.

Since the production of cell sheet does not require protein hydrolysis enzyme treatment, and the interactions between cells and the structure of cell sheet are well preserved on cell sheet, cell sheet for cell therapy tissue regeneration has become an emerging cell delivery method in recent years. The cell sheet technology has a higher cell concentration and more uniform cell distribution, and thus increasing the tissue regeneration capacity. In addition, the cells are stimulated by the culture medium and substrate in the cell sheet production, and can be induced to form cells with different characteristics. This phenomenon is particularly evident in mesenchymal stem cells (MSCs), which have the function of replacing or restoring damaged tissues. Guiding stem cells toward differentiated cells with specific function at the early stage of cellular sheet construction and maintaining these cellular characteristics is the most challenging cell culture procedure at present.

Currently, two main approaches are used to induce cell differentiation, one is to induce cell differentiation by chemical stimulation, and the other is to induce cell differentiation by physical mechanical stimulation or by stimulation of the surface structure of the substrate to which the cells are attached. Since chemical stimulation is difficult to control due to the concentration gradient and action time of the formula used, the biomimetic environment made with physical characteristics of mechanical stimulation and the surface structure of the substrate has better control effects and has a wider range of applications.

Mechanical stimulation and substrate surface structural stimulation have a decisive effect on the differentiation of mesenchymal stem cells. Therefore, in order to optimize and guide the pre-differentiation of stem cells to specific tissue cell characteristics, there is a strong need for a novel substrate that can provide both mechanical stimulation and substrate surface structural stimulation to induce cell differentiation.

CN 107 325 307 A discloses a polylactic acid hybrid biological membrane as well as a preparation method and an application thereof. The polylactic acid hybrid biological membrane is made of polylactic acid and collagen in the mass ratio being (1-9):(1:9), the polylactic acid hybrid biological membrane is 0.05-0.2 mm thick, the thickness precision is controlled to be +/-0.02 mm, the specific surface area is 0.001-0.01 m<2>/g, and the membrane has micropores with the largest pore diameter smaller than or equal to 10 mu m and has the tensile strength larger than or equal to 3 MPa. The polylactic acid hybrid biological membrane is made of polylactic acid and collagen, overcomes the defects of high degradation speed, unstable degradation time and fast absorption of collagen and poor adhesion of polylactic acid, is a novel anti-adhesion membrane, has a unique micropore structure, relatively large specific surface area and soft texture and can promote adhesion and proliferation of cells and prevent adhesion without impeding transfer of nutrients.

### SUMMARY

The invention is defined in the independent claims. Preferred embodiments of the invention are defined in the dependent claims. The present disclosure provides a non-fibrous film of which the composition comprises: a collagen; and a polyester polymer. A content of the polyester polymer in the non-fibrous film is 1-60 wt%. Moreover, the non-fibrous film has a swelling rate of 1-200 $\mu$m/hour or a swelling proportion per unit time of 0.1-2%/hour in an aqueous liquid.

The non-fibrous film is prepared by a method comprising (a) preparing a mixture solution; and (b) drying the mixture solution in a fume hood to form a film. A solute of the mixture solution comprises: a collagen and a polyester polymer, wherein a content of the polyester polymer in the non-fibrous film is 1-60 wt%. A solvent of the mixture solution comprises a perfluorocarbon solvent. Moreover, the non-fibrous film has a swelling rate of 1-200 $\mu$m/hour or a swelling proportion per unit time of 0.1-2%/hour in an aqueous liquid.

The present disclosure also provides a cell sheet comprising any non-fibrous film mentioned above; and at least one cell layer on the non-fibrous film. The cell layer is composed of a plurality of cells, and the plurality of cells is arranged in the same direction.

A detailed description is given in the following embodiments with reference to the accompanying drawings.

## BRIEF DESCRIPTION OF DRAWINGS

The present invention can be more fully understood by reading the subsequent detailed description and examples with references made to the accompanying drawings, wherein:

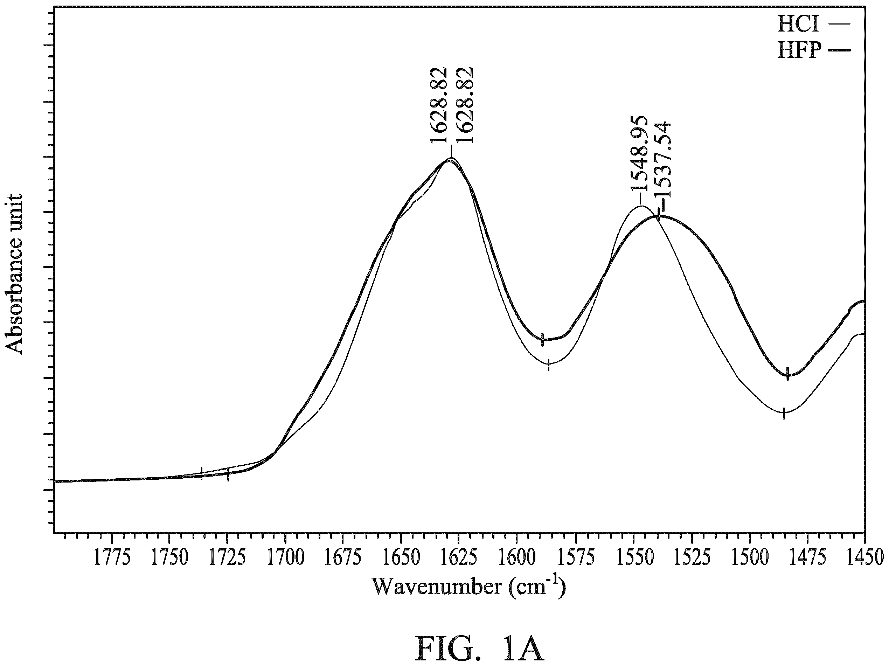

FIG. 1A shows the results of Fourier transform infrared spectroscopy (FTIR) analysis of the non-fibrous film prepared by Preparation example 1 (100 wt% collagen (hexafluoroisopropanol used as a solvent in the preparation thereof)) and the non-fibrous film prepared by Comparative preparation example 1 (100 wt% collagen (an acidic aqueous solution used as a solvent in the preparation thereof));

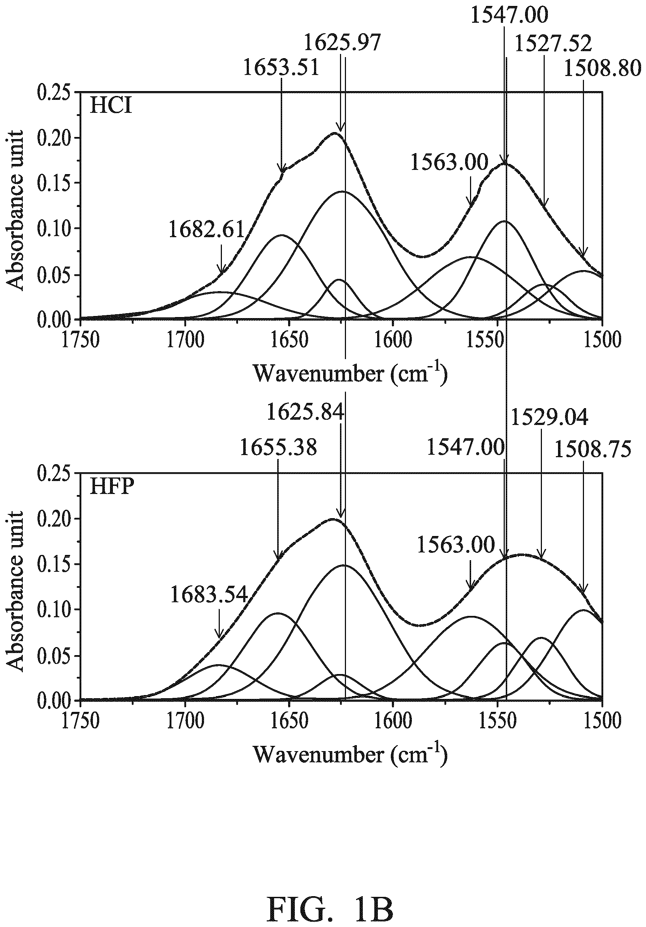

FIG. 1B shows the results of fitting the Fourier transform infrared spectra of the non-fibrous film prepared by Preparation example 1 (100 wt% collagen (hexafluoroisopropanol used as a solvent in the preparation thereof)) and the non-fibrous film prepared by Comparative preparation example 1 (100 wt% collagen (an acidic aqueous solution used as a solvent in the preparation thereof));

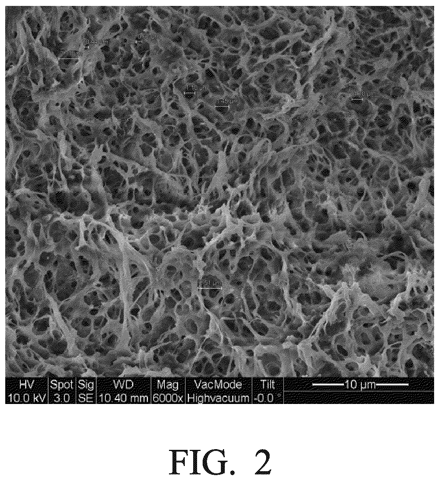

FIG. 2 shows a scanning electron microscopy photograph of the non-fibrous film prepared by Preparation example 8 (50 wt% collagen and 50 wt% polycarprolactone).

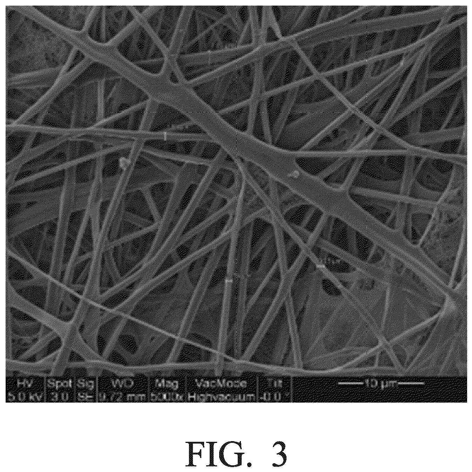

FIG. 3 shows a scanning electron microscopy photograph of the electrospun film (100 wt% of collagen) prepared by Comparative preparation example 3;

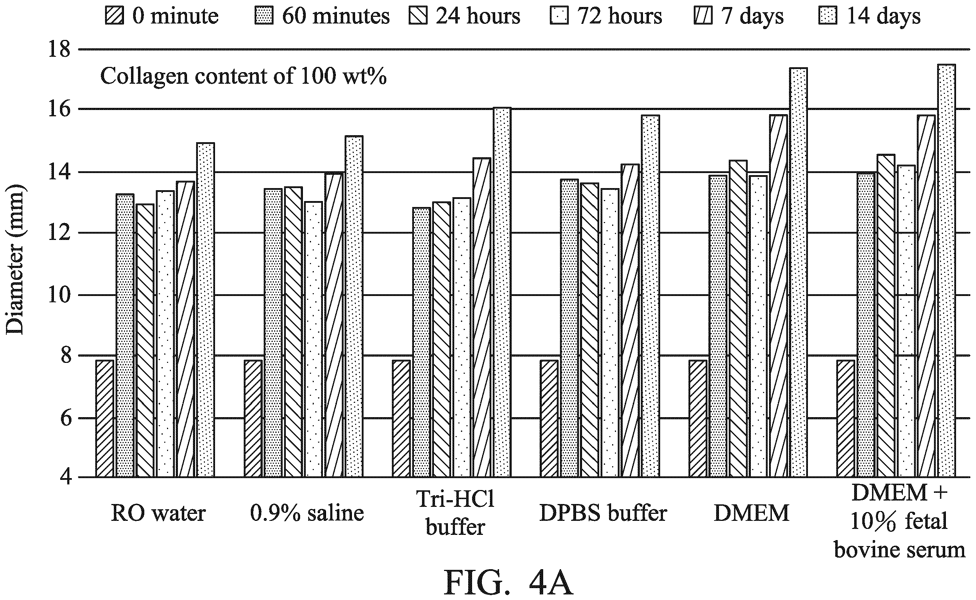

FIG. 4A shows the film diameters of the non-fibrous film prepared in Preparation example 1 (100 wt% collagen) determined in RO pure water, 0.9% saline, 10 mM Tri-HCl buffer, Dulbecco's phosphate buffered saline (DPBS), Dulbecco's Modified Eagle Medium (DMEM) or Dulbecco's Modified Eagle Medium (DMEM) containing 10% fetal bovine serum (FBS) (cell culture medium) at different time points in the film swelling test;

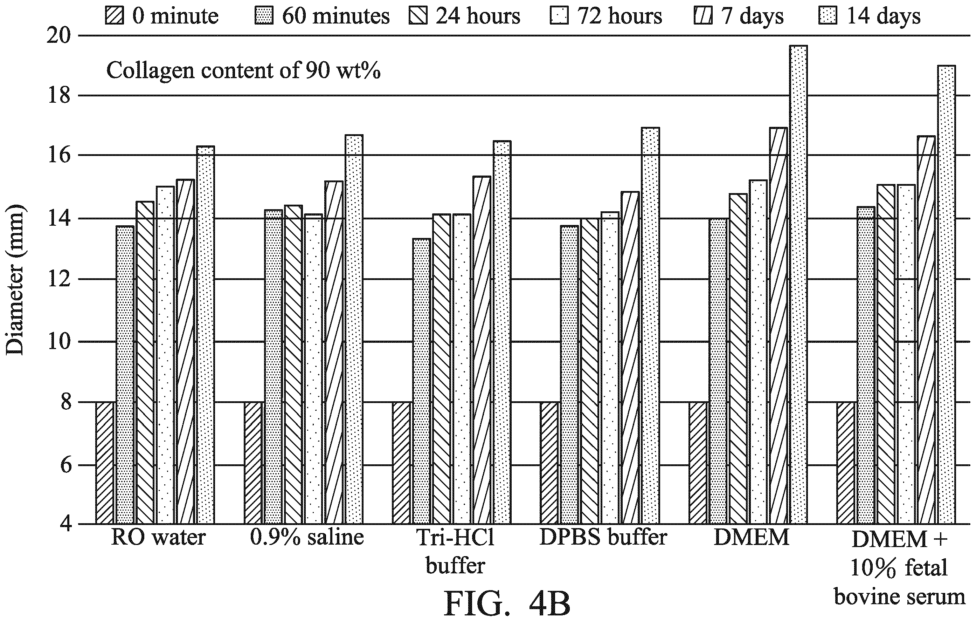

FIG. 4B shows he film diameters of the non-fibrous film prepared by Preparation example 2 (90 wt% collagen and 10 wt% polycarprolactone) determined in RO pure water, 0.9% saline, 10 mM Tri-HCl buffer, Dulbecco's phosphate buffered saline (DPBS), Dulbecco's Modified Eagle Medium (DMEM) or Dulbecco's Modified Eagle Medium (DMEM) containing 10% fetal bovine serum (FBS) (cell culture medium) at different time points in the film swelling test;

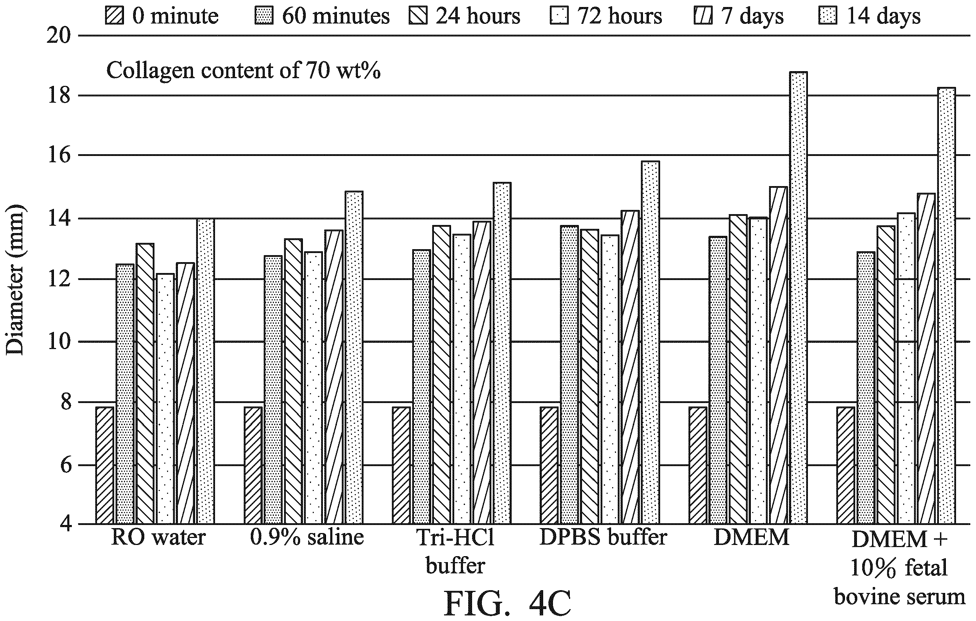

FIG. 4C shows he film diameters of the non-fibrous film prepared by Preparation example 7 (70 wt% collagen and 30 wt% polycarprolactone) prepared in Preparation example 7 determined in RO pure water, 0.9% saline, 10 mM Tri-HCl buffer, Dulbecco's phosphate buffered saline (DPBS), Dulbecco's Modified Eagle Medium (DMEM) or Dulbecco's Modified Eagle Medium (DMEM) containing 10% fetal bovine serum (FBS) (cell culture medium) at different time points in the film swelling test;

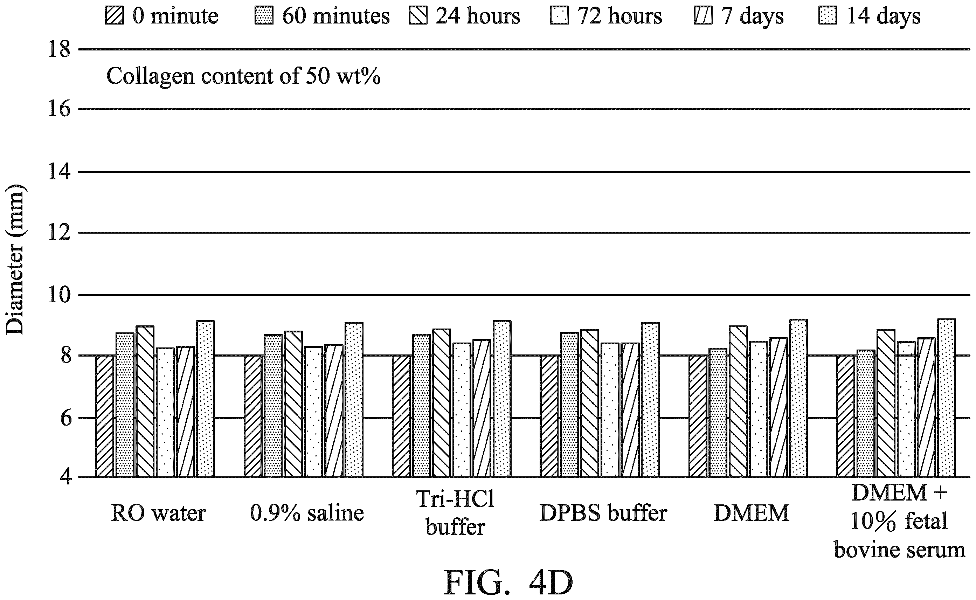

FIG. 4D shows he film diameters of the non-fibrous film prepared by Preparation example 8 (non-fibrous film: 50 wt% collagen and 50 wt% polycarprolactone) determined in RO pure water, 0.9% saline, 10 mM Tri-HCl buffer, Dulbecco's phosphate buffered saline (DPBS), Dulbecco's Modified Eagle Medium (DMEM) or Dulbecco's Modified Eagle Medium (DMEM) containing 10% fetal bovine serum (FBS) (cell culture medium) at different time points in the film swelling test;

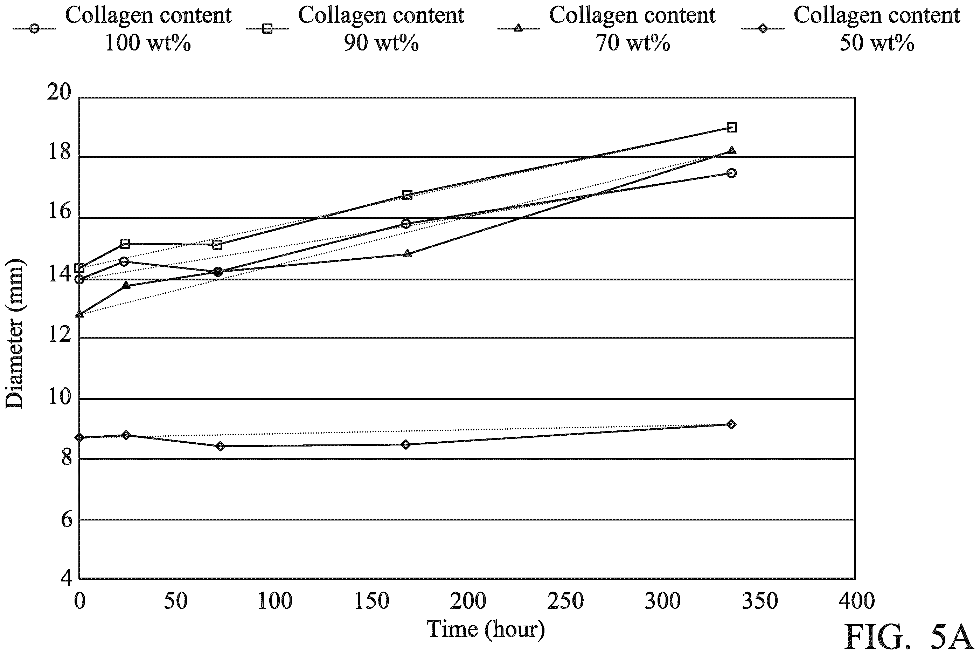

FIG. 5A shows the film diameters of the non-fibrous film prepared by Preparation example 1 (100 wt% collagen), the non-fibrous film prepared by Preparation example 2 (90 wt% collagen and 10 wt% polycarprolactone), the non-fibrous film prepared by Preparation example 7 (70 wt% collagen and 30 wt% polycarprolactone) prepared in Preparation example 7, and the non-fibrous film prepared by Preparation example 8 (non-fibrous film: 50 wt% collagen and 50 wt% polycarprolactone) in Dulbecco's Modified Eagle Medium (DMEM) containing 10% fetal bovine serum (FBS) (cell culture medium) determined at different time points in the film swelling test;

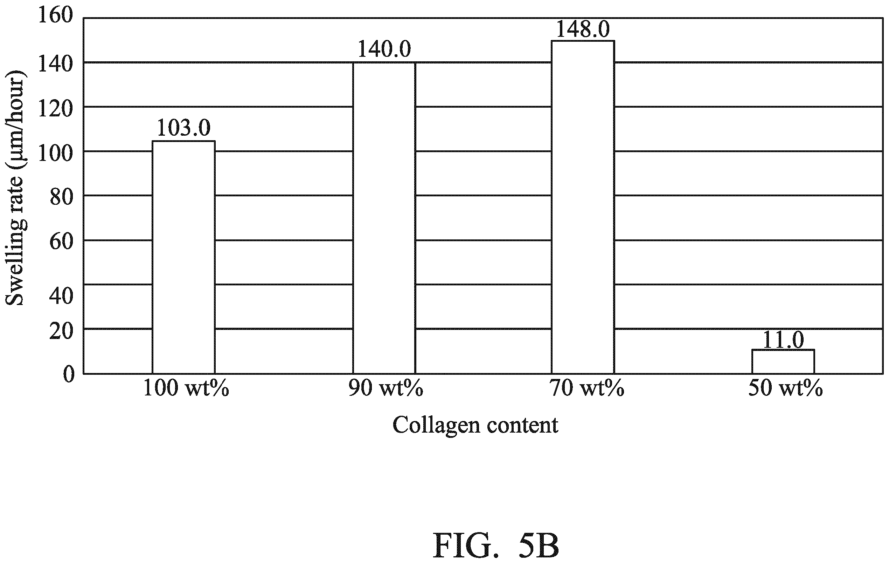

FIG. 5B shows the swelling rates of the non-fibrous film prepared by Preparation example 1 (100 wt% collagen), the non-fibrous film prepared by Preparation example 2 (90 wt% collagen and 10 wt% polycarprolactone), the non-fibrous film prepared by Preparation example 7 (70 wt% collagen and 30 wt% polycarprolactone) prepared in Preparation example 7, and the non-fibrous film prepared by Preparation example 8 (non-fibrous film: 50 wt% collagen and 50 wt% polycarprolactone) in Dulbecco's Modified Eagle Medium (DMEM) containing 10% fetal bovine serum (FBS) (cell culture medium) determined at different time points in the film swelling test;

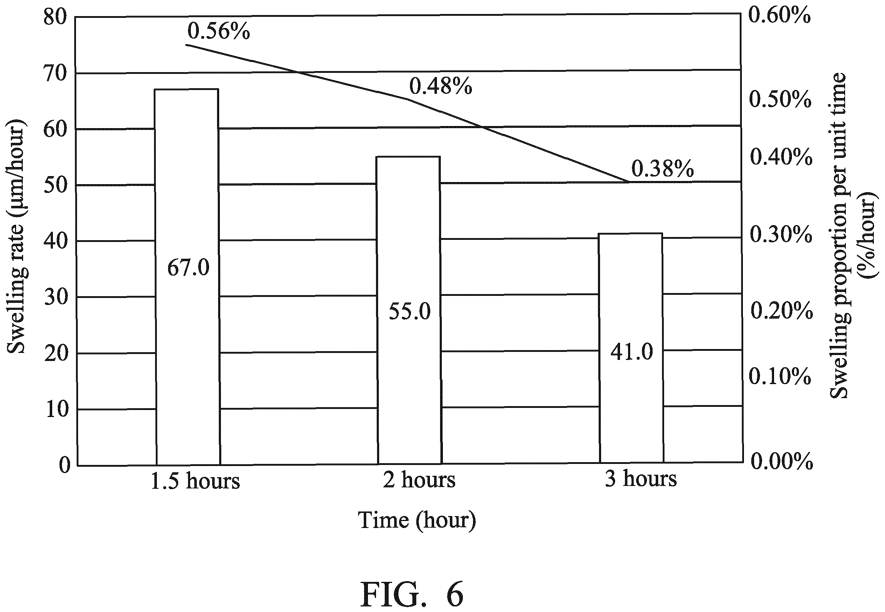

FIG. 6 shows the swelling rates and swelling proportion per unit time of the non-fibrous film prepared by Preparation example 4 (80 wt% collagen and 20 wt% polycarprolactone, crosslinking for 1.5 hours), the non-fibrous film prepared by Preparation example 5 (80 wt% collagen and 20 wt% polycarprolactone, crosslinking for 2 hours) and the non-fibrous film prepared by Preparation example 6 (80 wt% collagen and 20 wt% polycarprolactone, crosslinking for 3 hours) in DMEM solution containing 10% fetal bovine serums;

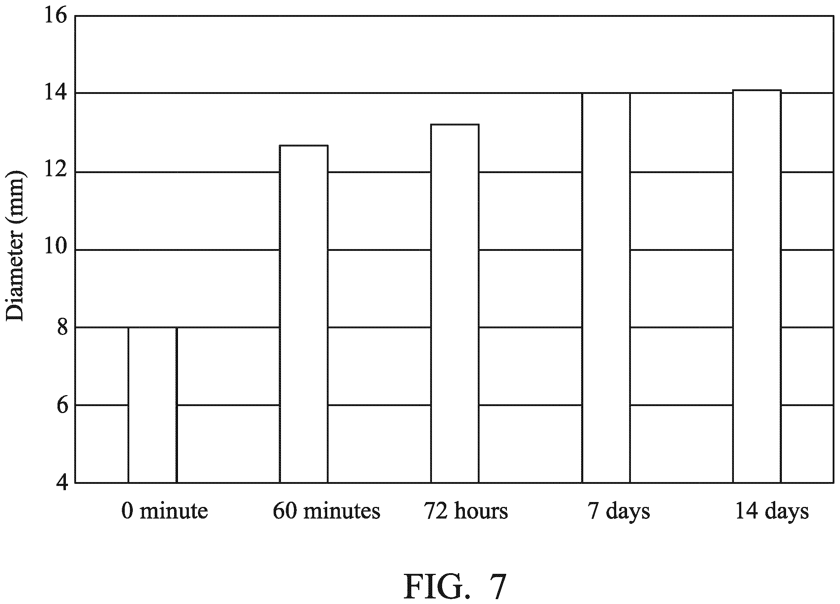

FIG. 7 shows the film diameters of the non-fibrous film prepared in preparation example 9 (90 wt% collagen and 10 wt% polylactic acid) determined in a PBS buffer at different time points in the film swelling test;

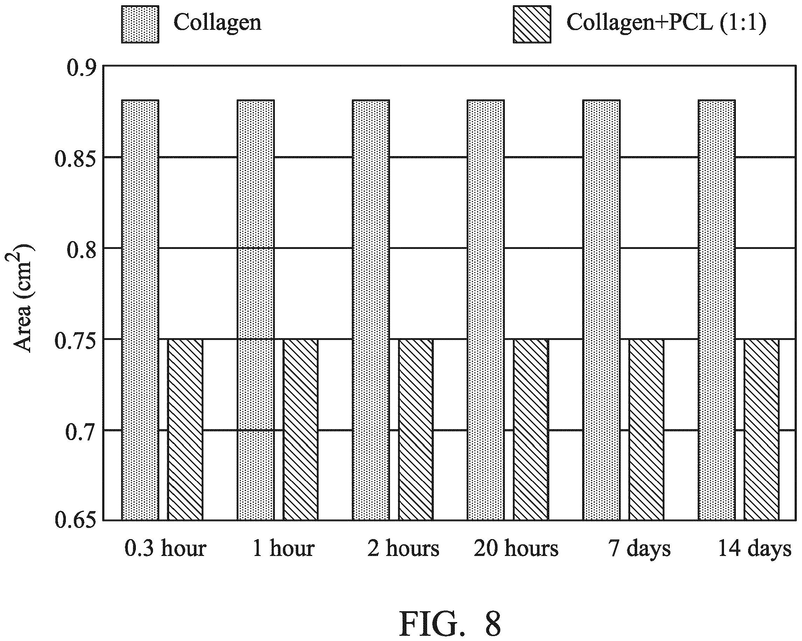

FIG. 8 shows the film area of the electrospun film prepared in Comparative preparation example 3 (100 wt% collagen) and the electrospun film prepared in Comparative preparation example 4 (50 wt% collagen and 50 wt% polycarprolactone) determined in PBS buffer at different time points in the film swelling test;

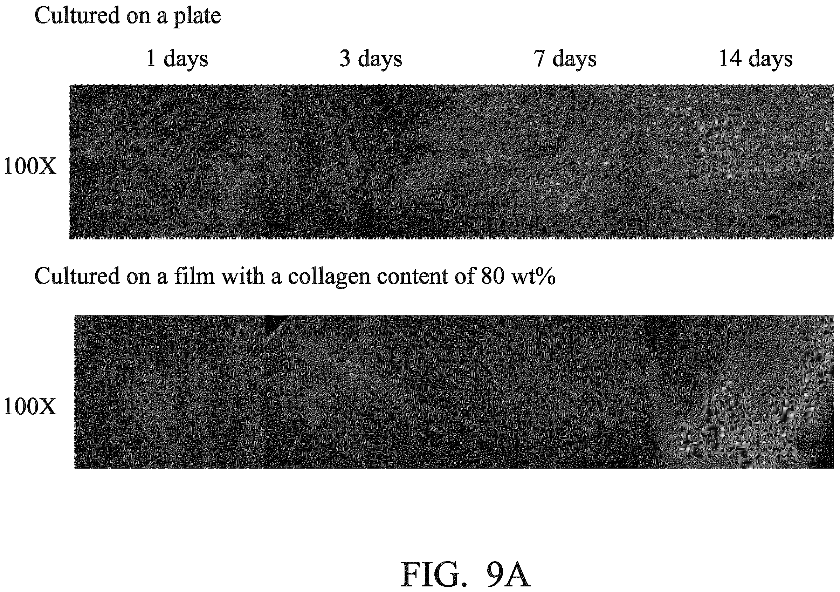

FIG. 9A shows photographs of the growth status of cells on the non-fibrous film prepared in prepared in Preparation example 3 (80 wt% collagen and 20 wt% polycarprolactone) and cells on the culture plate on Day 1, 3, 7 and 14 in the cell culture test;

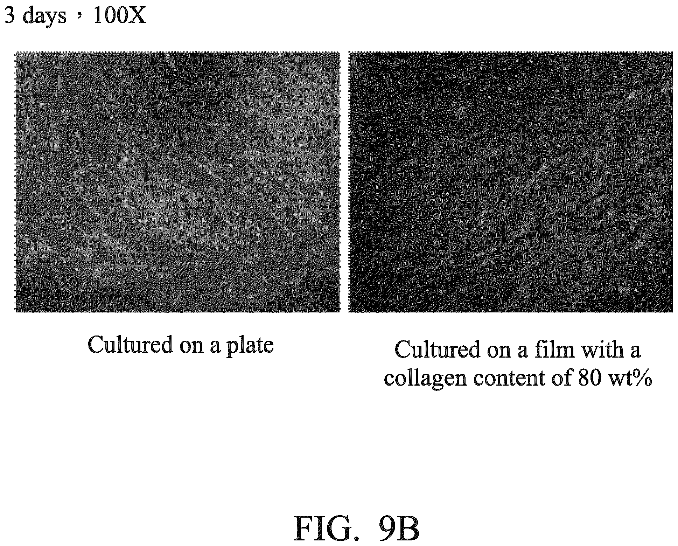

FIG. 9B shows photographs of the growth status of cells on the non-fibrous film prepared in prepared in Preparation example 3 (80 wt% collagen and 20 wt% polycarprolactone) and cells on the culture plate on Day 3 in the cell culture test; and

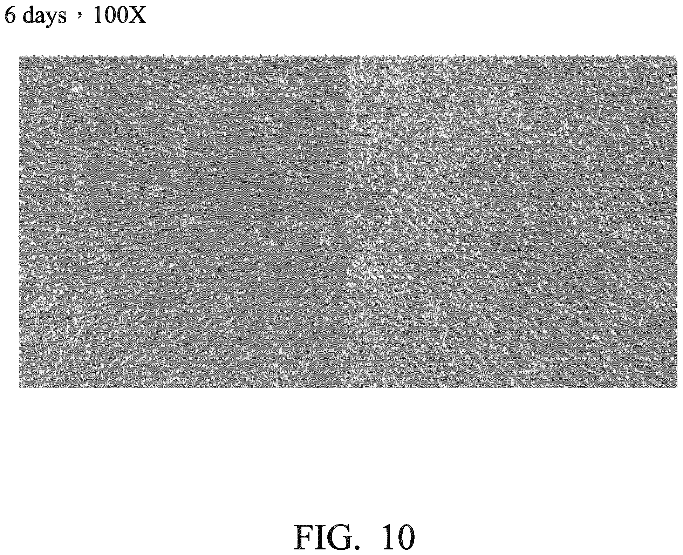

FIG. 10 shows a photograph of the growth status of cells on the non-fibrous film prepared in prepared in Comparative preparation example 2 (90 wt% collagen and 10 wt% polycarprolactone (an acidic aqueous solution used as a solvent)) on Day 6 in the cell culture test.

## DETAILED DESCRIPTION

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

The present disclosure provides a non-fibrous film which may be a biodegradable film, and this film has dynamic changes in size over time in an aqueous liquid. There is no particular limitation for the aqueous liquid mentioned above, as long as it does not result in adverse effects on the film. Examples of the aqueous fluids may include, but are not limited to, water, a buffer, and a cell culture medium.

The non-fibrous film of the present disclosure mentioned above may have cell-bearing capabilities and may be applied to cell culture. When the non-fibrous film of the present disclosure is applied to cell culture, since its size change dynamically in a cell culture medium, during cell culture, it can provide the cells which are attached thereto to grow with a physical mechanical force, such as a tensile stress, and a stimulus of structural changes on the surface to which the cells are attached, without the need of providing an additional mechanical and/or chemical stimulus to the film and/or cells, to allow the cells which are attached thereto to grow in the same direction and to promote cell differentiation.

The non-fibrous film of the present disclosure mentioned above is formed by a method as defined above.

First, a mixture solution is prepared.

The concentration of the solute in the prepared mixture solution is not particularly limited and can be adjusted as needed, as long as it does not adversely affect the formation of the film. For example, the concentration of solute in the mixture solution prepared can be adjusted according to the environment at the time of preparing the mixture solution (e.g., temperature, humidity, pressure of the environment), the type of solvent used, the environment at the time of subsequent film formation (e.g., temperature, humidity, pressure, etc.), and the method for film formation to be used, but is not limited thereto. For example, the concentration of the solute in the mixture solution may be about 0.5-20 wt%, such as about 0.5 wt%, about 0.8 wt%, about 1 wt%, about 3 wt%, about 5 wt%, 6 wt%, about 7 wt%, about 8 wt%, about 9 wt%, about 10 wt%, about 11 wt%, about 12 wt%, about 13 wt%, about 14 wt%, about 15 wt%, about 16 wt%, about 17 wt%, about 18 wt%, about 19 wt%, about 20 wt%, but it is not limited thereto.

Furthermore, the solute in the mixture solution mentioned above comprises a collagen and a polyester polymer.

The content of the polyester polymer in the solute mentioned above is 1-60 wt%, such as about 1-55 wt%, about 1-50 wt%, about 10-50 wt%, about 5 wt%, about 10 wt%, about 15 wt%, about 20 wt%, about 25 wt%, about 30 wt%, about 35 wt%, about 40 wt%, about 45 wt%, about 50 wt%, about 55 wt%, 60 wt%, but it is not limited thereto.

Moreover, in the solute mentioned above, a weight ratio of the collagen to the polyester polymer may be about 0.6-99:1, such as about 95:5, about 90:10, about 85:15, about 80:20, about 75:25, about 70:30, about 65:35, about 60:40, about 55:45, about 50:50, about 45:55, about 40:60, but it is not limited thereto.

In one embodiment, the solute mentioned above may consist of collagen and polyester polymer, and the weight ratio of the collagen to the polyester polymer may be about 0.6-99:1, such as about 95:5, about 90:10, about 85:15, about 80:20, about 75:25, about 70:30, about 65:35, about 60:40, about 55:45, about 50:50, about 45:55, about 40:60, but it is not limited thereto.

The collagen in the solute mentioned above has no particular limitations, and may include any type of collagen. Example of the collagen mentioned above may comprise at least one of the following: type I collagen, type II collagen and type III collagen, but it is not limited thereto. In one embodiment, the collagen mentioned above is type I collagen.

The molecular weight of the polyester polymer in the solute mentioned above may depend on the need, and has no particular limitation. For example, the molecular weight of the polyester polymer mentioned above may be about 50,000-1,500,000 Da, such as medium molecular weight (about 50,000-200,000 Da), high molecular weight (about 800,000-1,500,000 Da), about 50,000 Da, about 60,000 Da, about 70,000 Da, about 80,000 Da, about 90,000 Da, about 100,000 Da, about 150,000 Da, about 200,000 Da, about 250,000 Da, about 300,000 Da, about 400,000, about 500,000 Da, about 800,000 Da, about 1,000,000 Da, about 1,500,000 Da, but it is not limited thereto. In one embodiment, the molecular weight of the polyester polymer may be about 80,000 Da.

Furthermore, the intrinsic viscosity of the polyester polymer in the solute mentioned above may also depend on the need, and has no particular limitation. For example, the intrinsic viscosity of the polyester polymer mentioned above may be about 0.15-7 dl/g, such as about 0.15-0.3 dl/g, about 0.35-0.45 dl/g, about 0.8-1.2 dl/g, about 1.3-1.6 dl/g, about 1.6-2.4 dl/g, about 2.0-2.8 dl/g, about 3.5-5.5 dl/g. In one embodiment, the intrinsic viscosity of the polyester polymer mentioned above may be about 4.5 dl/g.

In addition, the type of the polyester polymer in the solute mentioned above also has no particular limitation, as long as it is biodegradable. Example of the polyester polymer may comprise, but is not limited to, polycarprolactone (PCL), polylactic acid (PLA), polyglycolic acid (PGA), polyhydroxybutyrate (PHB), polyhydroxyvalerate (PHV), or any combination thereof. In one embodiment, the polyester polymer in the solute mentioned above may be polycarprolactone. In another embodiment, the polyester polymer in the solute mentioned above may be polylactic acid.

A solvent of the mixture solution comprises a perfluorocarbon solvent. Examples of the perfluorocarbon solvent mentioned above may include, but are not limited to, hexafluoroisopropanol, hexafluoroacetone, 1,1,1-trichlorotrifluoroacetone, trifluoroacetic acid, perfluoroperhydrophenanthrene, perfluoro(methylcyclohexane), or any combination thereof. The solvent mentioned above may be used as a co-solvent for the collagen and the polyester polymer in the solute mentioned above, and can allow the collagen and the polyester polymer in the mixture solution mentioned above uniformly distributed. In addition, the solvent mentioned above can make collagen exist in the mixture solution mentioned above and the formed film in both the native form and the denatured form at the same time. In one example, the solvent of the mixture solution is hexafluoroisopropanol.

Next, after the mixture solution mentioned above is prepared, the mixture solution mentioned above is dried in a fume hood to form a film to obtain the non-fibrous film of the present disclosure.

In one embodiment, the mixture solution mentioned above can be poured into a mold and then dried to form a film. In another embodiment, the mixture solution mentioned above can be poured on a flat plate, scraped by a scraper, and then dried to form a film.

In one embodiment, the obtained non-fibrous film may be a porous film. In this embodiment, the content of the polyester polymer in the above-mentioned solute mentioned above may be about 45-60 wt%, such as about 45 wt%, about 50 wt%, about 55 wt%, 60 wt%, but it is not limited thereto. In this embodiment, the pore size of the pores of the porous film may be about 1-50 $\mu$m, such as about 5-50 $\mu$m, about 10-50 $\mu$m, about 15-50 $\mu$m, about 1-45 $\mu$m, about 1-40 $\mu$m, about 1-30 $\mu$m, about 1 $\mu$m, about 5 $\mu$m, about 10 $\mu$m, about 15 $\mu$m, about 20 $\mu$m, about 25 $\mu$m, about 30 $\mu$m, about 35 $\mu$m, about 40 $\mu$m, about 45 $\mu$m, about 50 $\mu$m, but it is not limited thereto.

In the foregoing embodiment where the obtained non-fibrous film may be a porous film, in the method for preparing the non-fibrous film of the present disclosure mentioned above, the solute of the mixture solution prepared may consist of collagen and polyester polymer. The polyester polymer may include at least one of the following: polycarprolactone, polylactic acid, polyglycolic acid, polyhydroxybutyrate, polyhydroxyvalerate, etc., but is not limited thereto. In one specific embodiment, when the obtained non-fibrous film is a porous film, in the method of preparing the non-fibrous film of the present disclosure mentioned above, the solute of the mixture solution consists of the collagen and the polyester polymer, and the polyester polymer is polycarprolactone. Moreover, in this specific embodiment, the molecular weight of the polycarprolactone may be about 50,000-200,000 Da, such as about 80,000 Da, but is not limited thereto.

In another embodiment, the obtained non-fibrous film may be a non-porous film. In this embodiment, the content of the polyester polymer in the solute mentioned above may be 1 wt% or more but less than 45 wt%, such as about 1-40 wt%, about 5-40 wt%, about 10-40 wt%, about 15-35 wt%, about 20-30 wt%, 1 wt%, about 3 wt%, about 5 wt%, about 10 wt%, about 15 wt%, about 20 wt%, about 25 wt %, about 30 wt%, about 35 wt%, about 40 wt%, about 44 wt%, but not limited thereto.

In the foregoing embodiment where the obtained non-fibrous film may be a non-porous film, in the method for preparing the non-fibrous film of the present disclosure mentioned above, the solute of the mixture solution prepared can be made of collagen and polyester. The polyester polymer may include at least one of the following: polycarprolactone, polylactic acid, polyglycolic acid, polyhydroxybutyrate, polyhydroxyvalerate, etc., but is not limited thereto. In one specific embodiment, when the obtained non-fibrous film is a non-porous film, in the method of preparing the non-fibrous film of the present disclosure mentioned above, the solute of the prepared mixture solution consists of the collagen and the polyester polymer, and the polyester polymer is polylactic acid. In this specific embodiment, the intrinsic viscosity of the polylactic acid may be about 0.15-7.0 dl/g, such as about 4.5 dl/g, but is not limited thereto.

Furthermore, in one embodiment, the method for preparing the non-fibrous film of the present disclosure mentioned above, in addition to the step of preparing a mixture solution and the step of drying the foregoing mixture solution to form a film mentioned above, may also further comprise after drying the foregoing mixture solution to form a film, performing a cross-linking treatment on the formed film to form a cross-linked non-fibrous film.

The time for the foregoing cross-linking treatment to the film has no particular limitation, and can be adjusted as needed. For example, the treatment time for the cross-linking treatment to the film can be adjusted based on the environment at the time of performing the cross-linking treatment (e.g., temperature, humidity, pressure of the environment), the constituent components and/or content of the components of the film, the cross-linking method or the cross-linking agent used, the desired film strength, the use of the film, etc., but it is not limited thereto. For example, treatment time for the foregoing cross-linking treatment to the film may be about 0.1-10 hours, such as about 0.1 hour, about 0.5 hour, about 1 hour, about 1.5 hours, about 2 hours, about 2.5 hours, about 3 hours, about 5 hours, about 6 hours, about 8 hours, about 10 hours, etc., but not limited thereto.

In addition, the cross-linking treatment to the film mentioned above may be performed with a cross-linking agent, but it is not limited thereto. Examples of the cross-linking agent mentioned above may include formaldehyde, glutaraldehyde, glyoxal, malondialdehyde, succinyl dialdehyde, phthalaldehyde, dialdehyde starch, polyacrolein, polymethacrolein, and any combination thereof, but they are not limited thereto.

In one embodiment, the cross-linking treatment to the film mentioned above may be performed with formaldehyde, such as 10% formaldehyde vapor, and the treatment time for the cross-linking treatment to the film may be about 0.1-10 hours, such as about 0.1 hour, about 0.5 hour, about 1 hour, about 1.5 hours, about 2 hours, about 2.5 hours, about 3 hours, about 5 hours, about 6 hours, about 8 hours, about 10 hours, but it is not limited thereto.

According to the foregoing, it is known that in the method for preparing the non-fibrous film of the present disclosure mentioned above, the solvent of the mixture solution can make collagen exist in the mixture solution and the formed film in both the native form and the denatured form at the same time. Since both natural collagen and denatured collagen exist in the formed film at the same time, when the non-fibrous film is in contact with a liquid, the natural collagen and denatured collagen molecules in the film due to hydration force have a competitive effect with each other in terms of force with the polyester polymer to allow the film has physical property of dynamic swelling or stretching.

Namely, as mentioned above, the non-fibrous film of the present disclosure prepared by any method described above has a dynamic change in size over time in a liquid.

The degree of dynamic changes in size of the non-fibrous film of the present disclosure in a liquid, such as the degree of swelling or stretching, may be adjusted by selecting different constituent components and/or contents of the components of the film for the solute of the mixture solution, or by adjusting the time for the cross-linking treatment when the film undergoes a cross-linking treatment, in the method for preparing the non-fibrous film of the present disclosure mentioned above, but it is not limited thereto. For example, the degree of dynamic changes in size of the non-fibrous film of the present disclosure in a liquid can be adjusted by adjusting the weight ratio of the collagen to the polyester polymer in the solute of the mixture solution, by adjusting the type and molecular weight/intrinsic viscosity of the polyester polymer in the solute of the mixture solution, and/or by adjusting the time of the cross-linking treatment when the film undergoes a cross-linking treatment.

The non-fibrous film of the present disclosure prepared by any method described above has a swelling rate of 1-200 $\mu$m/hour in an aqueous liquid, such as about 5-200 $\mu$m/hour, about 10-200 $\mu$m/hour, about 10-150 $\mu$m/hour, 1 $\mu$m/hour, about 2 $\mu$m/hour, about 5 $\mu$m/hour, about 10 $\mu$m/hour, about 11 $\mu$m/hour, about 100 $\mu$m/hour, about 103 $\mu$m/hour, about 110 $\mu$m/hour, about 120 $\mu$m/hour, about 130 $\mu$m/hour, about 140 $\mu$m/hour, about 148 $\mu$m/hour, about 150 $\mu$m/hour, 200 $\mu$m/hour, but not limited thereto. In another embodiment, the non-fibrous film of the present disclosure prepared by any method described above has a swelling proportion per unit time of 0.1-2%/hour in an aqueous liquid, such as about 0.1-1.5%/hour, about 0.1-1%/hour, about 0.1-0.5%/hour, about 0.1-0.2%/hour, about 0.2-2%/hour, about 0.5-2%/hour, about 0.1-2%/hour, about 0.15-2%/hour, 0.1%/hour, about 0.2%/hour, about 0.5%/hour, about 1%/hour, about 1.5%/hour, 2%/hour, but not limited thereto

In addition, the present disclosure may also provide a method for culturing cells, which may include, but is not limited to, culturing cells on any non-fibrous film of the present disclosure mentioned above in a culture medium.

Since any non-fibrous film of the present disclosure mentioned above may have cell-bearing capabilities, and its size changes dynamically in a culture medium, during cell culture, it can continuously provide the cells which are attached thereon to grow with a physical mechanical force, such as a tensile stress, and a stimulus of structural changes of the surface to which the cells are attached, without the need of providing an additional mechanical and/or chemical stimulus to the film and/or cells, to allow the cells which are attached thereto to grow in the same direction and/or to promote cell differentiation.

The cells used in the method for culturing cells of the present disclosure can be selected as needed and has no particular limitation. For example, the type of cell can be selected according to the desired application field, but it is not limited thereto. In one embodiment, the cells need to be used for tissue regeneration in a living body, and thus cells with differentiation ability can be used in the method of culturing cells of the present disclosure mentioned above. Examples of cells with differentiation ability may include, but are not limited to, fibroblast, myoblasts, epithelial cells, endothelial cells, progenitor cells, tenocytes, stem cells, mesenchymal stem cells, bone marrow stem cells, and adipose derived stem cells.

In the method for culturing cells of the present disclosure, the culture medium used for culturing the cells can be selected as needed and has no particular limitation. For example, the selection can be made according to the cell to be cultured, the state to be achieved by the cell, but it is not limited thereto.

In addition, in the method for culturing cells of the present disclosure, the time for culturing cells can be adjusted as needed, and has no particular limitation. For example, the time for culturing the cells can be adjusted according to the type of cells, the growth status of the cells, the environment in which the cells are cultured (such as the temperature and humidity of the environment), the culture medium used, and so on. In one embodiment, the time for culturing the cells may be about 1-28 days, such as about 1 day, about 1.5 days, about 2 days, about 3 days, about 5 days, about 6 days, about 7 days, about 10 days, about 14 days, about 21 days, about 28 days, but it is not limited thereto.

Furthermore, in the method for culturing cells of the present disclosure, the temperature of culturing cells can be adjusted as needed, and has no particular limitation. For example, the temperature of the cultured cells can be adjusted according to the type of cells, the growth status of the cells, the culture medium used, and so on. In one embodiment, the temperature for culturing the cells may be about 30-40°C, such as about 30°C, about 32°C, about 35°C, about 36°C, about 37°C, about 37.5°C, about 38°C, about 39°C, about 40°C, but it is not limited thereto.

Furthermore, in the method for culturing cells of the present disclosure, the cells mentioned above may grow into at least one layer of cells on a non-fibrous film. In other words, in the method of culturing cells of the present disclosure, the cells mentioned above may grow into a single layer of cells or grow into multiple layers of cells on the non-fibrous film.

In one embodiment, the method for culturing cells of the present disclosure may further comprise, after the cells mentioned above growing into at least one layer of cells on the non-fibrous film, culturing cells which are different type from that of the cells mentioned above in a culture medium which is the same as or different from the foregoing culture medium, on this non-fibrous film with at least one layer of cells, to allow different cells be able to grow into at least one layer of cells. This step can be repeated as needed, and each time this step is repeated, the selected cells are different from the previous one.

Regarding the selection and culture conditions of different cells, please refer to the foregoing paragraphs with regard to cell selection, culture medium selection, culturing time and culturing temperature, etc., and thus they are not repeated herein.

Moreover, similarly, the present disclosure may also provide a method for forming a cell sheet, which may comprise culturing cells in a culture medium on any non-fibrous film of the present disclosure mentioned above to allow the cells grow in the same direction on any non-fibrous film of the present disclosure mentioned above and form at least one layer of cells to form a cell sheet, but it is not limited thereto.

Since any non-fibrous film of the present disclosure mentioned above may have cell-bearing capabilities, and its size changes dynamically in a culture medium, during cell culture, it can continuously provide the cells which are attached thereon to grow with a physical mechanical force, such as a tensile stress, and a stimulus of structural changes of the surface to which the cells are attached, without the need of providing an additional mechanical and/or chemical stimulus to the film and/or cells, to allow the cells which are attached thereto to grow in the same direction and/or to promote cell differentiation to obtain a cell sheet which has at least one layer of cells of a plurality of cells arranged in the same direction.

In one embodiment, the method for forming a cell sheet of the present disclosure may further comprise, after the cells mentioned above growing into at least one layer of cells on the non-fibrous film, culturing cells which are different type from that of the cells mentioned above in a culture medium which is the same as or different from the foregoing culture medium, on this non-fibrous film with at least one layer of cells, to allow different cells be able to grow into at least one layer of cells. This step can be repeated as needed, and each time this step is repeated, the selected cells are different from the previous one.

Regarding the selection and culture conditions of different cells, please refer to the descriptions for cell selection, culture medium selection, culturing time and culturing temperature, etc. in the foregoing paragraphs with regard to the method for culturing cells of the present disclosure, and thus they are not repeated herein.

Furthermore, based on the above, the present disclosure also provides a cell sheet, which includes any non-fibrous film of the present disclosure mentioned above, and at least one layer of cells on any non-fibrous film of the present disclosure mentioned above, wherein the cell layer is composed of a plurality of cells, and the plurality of cells are arranged in the same direction. In one embodiment, the cells in the cell sheet of the present disclosure may have better differentiation ability.

In one embodiment, the cell sheet of the present disclosure may have multiple layers of cell layers, and the cells in each layer of cells may be the same or different.

The cells in the cell sheet of the present disclosure mentioned above may be selected as needed, and has no particular limitation. For example, the type of cell can be selected according to the desired field to which the cell sheet applied, but it is not limited thereto. In one embodiment, the cell sheet need to be used for tissue regeneration in a living body, and thus cells with differentiation ability can be used in the cell sheet of the present disclosure mentioned above. Examples of cells with differentiation ability may include, but are not limited to, fibroblast, myoblasts, epithelial cells, endothelial cells, progenitor cells, tenocytes, stem cells, mesenchymal stem cells, bone marrow stem cells, and adipose derived stem cells.

EXAMPLES

Example 1: Preparation of film

1. Preparation example 1 (Reference-example, not according to the claimed invention)

Non-fibrous film: 100 wt% of collagen

Type I collagen and hexafluoroisopropanol (HFP) as a solvent were uniformly mixed with to prepare a 10% (w/w) collagen solution.
15 g of this collagen solution was poured into a Teflon mold (a bottom dimension of which was 7 cm*7 cm) and placed in a fume hood to dry to form a film.
After drying to form the film, the film was removed from the mold and cross-linked with 10% formaldehyde for 1 hour. Afterwards, a cross-linked non-fibrous film with a collagen content of 100 wt% was obtained.

2. Preparation example 2

Non-fibrous film: 90 wt% collagen and 10 wt% polycarprolactone (PCL)

1.574 g of type I collagen and 0.17 g of polycarprolactone (Mw: 80,000 Da) were added to 17.9 g of hexafluoroisopropanol which was used as a solvent and uniformly mixed to prepare a mixture solution.
The mixture solution is poured into a Teflon mold (a bottom dimension of which was 7 cm*7 cm) and placed in a fume hood to dry to form a film.
After drying to form the film, the film was removed from the mold and cross-linked with 10% formaldehyde for 1 hour. Afterwards, a cross-linked non-fibrous film with a collagen content of 90 wt% was obtained.

3. Preparation example 3

Non-fibrous film: 80 wt% collagen and 20 wt% polycarprolactone

1.36 g of type I collagen and 0.34 g of polycarprolactone (Mw: 80,000 Da) were added to 16 g of hexafluoroisopropanol which was used as a solvent and uniformly mixed to prepare a mixture solution.
The mixture solution was poured into a Teflon mold (a bottom dimension of which was 7 cm*7 cm) and placed in a fume hood to dry to form a film.
After drying to form the film, the film was removed from the mold and cross-linked with 10% formaldehyde for 1 hour. Afterwards, a cross-linked non-fibrous film with a collagen content of 80 wt% was obtained.

4. Preparation example 4

Non-fibrous film: 80 wt% collagen and 20 wt% polycarprolactone, crosslinking for 1.5 hours

Type I collagen, polycarprolactone (Mw: 80,000 Da) and hexafluoroisopropanol as solvent were uniformly mixed to prepare a mixture solution with a solute concentration of 10% (w/w), wherein the weight ratio of type I collagen to polycarprolactone was 8:2.
The mixture solution was poured into a Teflon mold (a bottom dimension of which was 7 cm*7 cm) and placed in a fume hood to dry to form a film.
After drying to form the film, the film was removed from the mold and cross-linked with 10% formaldehyde for 1.5 hours. Afterwards, a cross-linked non-fibrous film (cross-linking time 1.5 hours) with a collagen content of 80 wt% was obtained.

5. Preparation example 5

Non-fibrous films: 80 wt% collagen and 20 wt% polycarprolactone, crosslinking for 2 hours.

The preparation conditions and steps were the same as those in Preparation example 4, except that the crosslinking time was adjusted to 2 hours.

6. Preparation example 6

Non-fibrous films: 80 wt% collagen and 20 wt% polycarprolactone, crosslinking for 3 hours

The preparation conditions and steps were the same as those in Preparation example 4, except that the crosslinking time was adjusted to 3 hours.

7. Preparation example 7

Non-fibrous film: 70 wt% collagen and 30 wt% polycarprolactone

2.38 g of type I collagen and 1.08 g of polycarprolactone (Mw: 80,000 Da) were added to 21.76 g of hexafluoroisopropanol which was used as a solvent and uniformly mixed to prepare a mixture solution.
The mixture solution was poured into a Teflon mold (a bottom dimension of which was 7 cm*7 cm) and placed in a fume hood to dry to form a film.
After drying to form the film, the film was removed from the mold and cross-linked with 10% formaldehyde for 1 hour. Afterwards, a cross-linked non-fibrous film with a collagen content of 70 wt% was obtained.

8. Preparation example 8

Non-fibrous film: 50 wt% collagen and 50 wt% polycarprolactone

2.34 g of type I collagen and 2.34 g of polycarprolactone (Mw: 80,000 Da) were added to 46.3 g of hexafluoroisopropanol which was used as a solvent and uniformly mixed to prepare a mixture solution.
The mixture solution was poured into a Teflon mold (a bottom dimension of which was 7 cm*7 cm) and placed in a fume hood to dry to form a film.
After drying to form the film, the film was removed from the mold and cross-linked with 10% formaldehyde for 1 hour. Afterwards, a cross-linked non-fibrous film with a collagen content of 50 wt% was obtained.

9. Preparation example 9

Non-fibrous film: 90 wt% collagen with 10 wt% polylactic acid (PLA)

4.61 g of type I collagen and 0.512 g of poly (D-lactic acid) (PDLA) (4.5 dl/g) were added to 68.89 g of hexafluoroisopropanol which was used as a solvent and uniformly mixed to prepare a mixture solution.
69.15 g of this mixture solution was poured into a Teflon mold (a bottom dimension of which was 20 cm*20 cm) and placed in a fume hood to dry to form a film.
After drying to form the film, the film was removed from the mold and cross-linked with 10% formaldehyde for 1 hour. Afterwards, a cross-linked non-fibrous film with a collagen content of 90 wt% was obtained.

10. Preparation example 10

Non-fibrous film: 70 wt% collagen with 30 wt% polylactic acid

3.32 g of type I collagen and 1.42 g of poly (D-lactic acid) (PDLA) (4.5 dl/g) were added to 68.89 g of hexafluoroisopropanol which was used as a solvent and uniformly mixed to prepare a mixture solution.
69.15 g of this mixture solution was poured into a Teflon mold (a bottom dimension of which was 20 cm*20 cm) and placed in a fume hood to dry to form a film.
After drying to form the film, the film was removed from the mold and cross-linked with 10% formaldehyde for 1 hour. Afterwards, a cross-linked non-fibrous film with a collagen content of 70 wt% was obtained.

11. Comparative preparation example 1

Non-fibrous film: 100 wt% collagen (acidic aqueous solution as a solvent)

Type I collagen and an acidic aqueous solution (pH 3) as solvent were uniformly mixed to prepare a 1% (w/w) collagen solution.

The collagen solution was poured into a Teflon mold (a bottom dimension of which was 13 cm*13 cm) and placed in a fume hood to dry to form a film.

After drying to form the film, the film was removed from the mold and cross-linked with 10% formaldehyde for 1 hour. Afterwards, a cross-linked non-fibrous film with a collagen content of 100 wt% was obtained.

12. Comparative preparation example 2

Non-fibrous type film: 90 wt% collagen with 10 wt% polycarprolactone (acidic aqueous solution as a solvent)

Type I collagen and polyvinylpyrrolidone (K30) were added to an acidic aqueous solution (pH3) which was used as a solvent and uniformly mixed to prepare a mixture solution (concentration of type I collagen was 0.9% (w/w) and concentration of polyvinylpyrrolidone was 0.1 (w/w).

67 g of this mixture solution was poured into a Teflon mold (a bottom dimension of which was 13 cm*13 cm) and placed in a fume hood to dry to form a film.

After drying to form the film, the film was removed from the mold and cross-linked with 10% formaldehyde for 1 hour. Afterwards, a cross-linked non-fibrous film with a collagen content of 90 wt% was obtained.

13. Comparative preparation example 3

Electrospun film: 100 wt% of collagen

Type I collagen and hexafluoroisopropanol as a solvent were uniformly mixed to prepare a 10% (w/w) collagen solution.

The collagen solution was used as a raw material to electrospun by an electrospinning device (needle 25-26G; solution flow rate 0.15 mL/hour; voltage 5-6 kV) to form an electrospun film. The electrospun film manufactured was cross-linked with 10% formaldehyde for 1 hour. After that, a cross-linked electrospun film with a collagen content of 100 wt% was obtained.

14. Comparative preparation example 4

Electrospun films: 50 wt% collagen and 50 wt% polycarprolactone

Type I collagen, polycarprolactone (Mw: 80,000 Da) and hexafluoroisopropanol as a solvent were uniformly mixed to prepare a mixture solution with a solute concentration of 10% (w/w), wherein the weight ratio of type I collagen to polycarprolactone was 1: 1.

The collagen solution was used as a raw material to electrospun by an electrospinning device (needle 25-26G; solution flow rate 0.15 mL/hour; voltage 5-6 kV) to form an electrospun film. The electrospun film manufactured was cross-linked with 10% formaldehyde for 1 hour. After that, a cross-linked electrospun film with a collagen content of 50 wt% was obtained.

Example 2

Fourier-transform infrared spectroscopy (FTIR)

The non-fibrous film prepared by Preparation example 1 (100 wt% collagen (hexafluoroisopropanol was used as a solvent in the preparation thereof)) and the non-fibrous film prepared by Comparative preparation example 1 (100 wt% collagen (an acidic aqueous solution was used as a solvent in the preparation thereof)) were subjected Fourier-transform infrared spectroscopy (FTIR) analysis. The results are shown in FIG. 1A.

Also, Tables 1 and 2 show the structures represented by the peaks of collagen in Fourier transform infrared spectroscopy (based on Olena S. Rabotyagova et al, "Collagen structural hierarchy and susceptibility to degradation. susceptibility to degradation by ultraviolet radiation" Materials Science and Engineering C 28 (2008), pp. 1420-1429).

Table 1: Assignment of the band components of amide I region

| Position (cm$^{-1}$) | Assignment |
|---|---|
| 1690 | Intermolecular association. Helixes of aggregated collagen-like peptides β-turn |
| 1675 | Collagen in triple helix, with contribution from alpha-helix and beta-turns (protein amide stretching vibration of C=O) |
| 1660 | Random chain contribution of the non-proline and non-hydroxyproline |
| 1646-1650 | Random coil conformation: imide residues (amide I in random coil) Left-handed 3-10 helix in denatured state |
| 1630 | Partial beta-sheet region |

Table 2: Assignment of the band components of amide II region

| Position (cm$^{-1}$) | Assignment | Note |
|---|---|---|
| 1565 | Carboxyl groups N-H bending vibration and C-N stretching vibration | Side chains of D and E |
| 1549 | Amide II in triple helix | Collagen in triple helix |
| 1530 | Amide II in a random coil | Disordered structure due to degeneration |
| 1515 | Tyrosine side chain | Transformation of F to Y |

According to Tables 1 and 2, it is known that the peak at 1549 cm$^{-1}$ represents the characteristics of a triple helix of collagen, the peak at 1530 cm$^{-1}$ represents the loose structure of denatured collagen, and the peak at 1646-1650 cm$^{-1}$ and the peak at 1660 cm$^{-1}$ represent the collagen structure appearance of random coil.

Therefore, based on the results of FIG. 1A and Tables 1 and 2, it is known that compared to the non-fibrous film prepared by Comparative preparation example 1 (100 wt% collagen (an acidic aqueous solution was used as a solvent in the preparation thereof)), amide II band for the non-fibrous film prepared by Preparation example 1 (100 wt% collagen (hexafluoroisopropanol was used as a solvent in the preparation thereof)) significantly shifts from peak at 1548.9 cm$^{-1}$, where it was originally located, to the peak at 1537.5 cm$^{-1}$, indicating a decrease in the triple helix structure and an increase in the denatured collagen structure.

Moreover, the Fourier transform infrared spectra of the non-fibrous film prepared by Preparation example 1 (100 wt% collagen (hexafluoroisopropanol was used as a solvent in the preparation thereof)) and the non-fibrous film prepared by Comparative preparation example 1 (100 wt% collagen (an acidic aqueous solution was used as a solvent in the preparation thereof)) shown in FIG. 1A were fitted and the result are shown in FIG. 1B and Table 3.

Table 3

| Non-fibrous film prepared by Comparative preparation example 1 (an acidic aqueous solution was used as a solvent in the preparation thereof) | | | Non-fibrous film prepared by Preparation example 1 (hexafluoroisopropanol was used as a solvent in the preparation thereof) | | |
|---|---|---|---|---|---|
| Peak (cm$^{-1}$) | Area | Ratio (%) | Peak (cm$^{-1}$) | Area | Ratio (%) |
| 1508.8 | 1.219 | 6.57 | 1508.75 | 1.905 | 10.63 |
| 1527.52 | 0.847 | 4.57 | 1529.04 | 1.374 | 7.67 |
| 1547 | 2.954 | 15.94 | 1547 | 1.329 | 7.42 |
| 1563 | 2.857 | 15.41 | 1563 | 3.431 | 19.15 |
| 1624.02 | 5.915 | 31.91 | 1624.12 | 5.549 | 30.98 |
| 1625.97 | 0.682 | 3.68 | 1625.84 | 0.4784 | 2.67 |
| 1653.51 | 2.785 | 15.02 | 1655.38 | 2.784 | 15.54 |

(continued)

| Non-fibrous film prepared by Comparative preparation example 1 (an acidic aqueous solution was used as a solvent in the preparation thereof) | | | Non-fibrous film prepared by Preparation example 1 (hexafluoroisopropanol was used as a solvent in the preparation thereof) | | |
|---|---|---|---|---|---|
| Peak (cm$^{-1}$) | Area | Ratio (%) | Peak (cm$^{-1}$) | Area | Ratio (%) |
| 1682.61 | 1.277 | 6.89 | 1683.54 | 1.064 | 5.94 |

According to the results shown in Fig. 1B and Table 3 and Tables 1 and 2, it is known that compared to the non-fibrous film prepared by Comparative preparation example 1 (an acidic aqueous solution was used as a solvent in the preparation thereof), for the non-fibrous film prepared by Preparation example 1 (hexafluoroisopropanol was used as a solvent in the preparation thereof), area proportion of the peak at 1547 cm$^{-1}$ representing the characteristic of the collagen amide II triple helix reduces from about 15.94% to about 7.42%, the area proportion of the peak at 1527 cm$^{-1}$ representing the loose structure of collagen amide II after denaturation increase from about 4.57% to about 7.67%, and the area proportion of the peak at 1653 cm$^{-1}$ representing the collagen amide I random coil conformation increases from about 15.02% to about 15.54%.

Based on the foregoing, it is known that compared to the non-fibrous film prepared by Comparative preparation example 1 (an acidic aqueous solution was used as a solvent in the preparation thereof), the non-fibrous film prepared by Preparation example 1 (hexafluoroisopropanol was used as a solvent in the preparation thereof) has more denatured collagen, i.e., both natural collagen and denatured collagen are present in the non-fibrous film prepared by Preparation example 1 (hexafluoroisopropanol was used as a solvent in the preparation thereof) at the same time.

In other words, by using hexafluoroisopropanol as a solvent in the preparation of the collagen-containing film, the prepared film can have presence of both natural collagen and denatured collagen at the same time.

Example 3

Scanning electron microscopy (SEM)

The non-fibrous film prepared by Preparation example 8 (50 wt% collagen and 50 wt% polycarprolactone) and the electrospun film (100 wt% collagen) prepared by Comparative preparation example 3 were analyzed by scanning electron microscope, and the results are shown in FIG. 2 and FIG. 3, respectively.

FIG. 2 shows a scanning electron microscopy photograph of the non-fibrous film prepared by Preparation example 8 (50 wt% collagen and 50 wt% polycarprolactone). According to FIG. 2, it is known that the non-fibrous film prepared by Preparation example 8 (50 wt% collagen and 50 wt% polycarprolactone) is a porous non-fibrous film with a pore size of about 1 μm.

FIG. 3 shows a scanning electron microscopy photograph of the electrospun film (100 wt% of collagen) prepared by Comparative preparation example 3. According to FIG. 3, it is known that the electrospun film (100 wt% collagen) prepared by Comparative example 3 is a fibrous film.

Example 4

Film swelling test

The films prepared in the Preparation examples and the Comparative preparation examples were separately subjected to swelling tests.

1. Method

The film to be tested was cut by a circular cutter to obtain a circular film of 8 mm diameter.

The circular film was immersed in an aqueous solution (RO pure water, 0.9% saline, 10 mM Tri-HCl buffer, Dulbecco's phosphate buffered saline (DPBS), Dulbecco's Modified Eagle Medium (DMEM) or Dulbecco's Modified Eagle Medium (DMEM) containing 10% fetal bovine serum (FBS) (cell culture medium)).

When the film was immersed for 0 minute, 60 minutes, 24 hours, 72 hours, 7 days and 14 days, it was photographed by a microscope (NIKON model) at 0.75 magnification and the diameter thereof was measured.

The diameters for the film measured at the two time points were selected, and the swelling rate of the film could be calculated based on the following formula:

$$k=(y2-y1)/(x2-x1)$$

k is the swelling rate, x1 is the first time point, x2 is the second time point, y1 is the film diameter measured at the first time point, and y2 is the film diameter measured at the second time point.

2. Results

(1) With regard to the films prepared in Preparation example 1, 2, 3, 7 and 8

The film diameters of the non-fibrous film prepared by Preparation example 1 (100 wt% collagen), the non-fibrous film prepared by Preparation example 2 (90 wt% collagen and 10 wt% polycarprolactone), the non-fibrous film prepared by Preparation example 7 (70 wt% collagen and 30 wt% polycarprolactone) prepared in Preparation example 7, and the non-fibrous film prepared by Preparation example 8 (non-fibrous film: 50 wt% collagen and 50 wt% polycarprolactone) determined in different aqueous liquids at different time points are shown in FIG. 4A, FIG. 4B, FIG. 4C and FIG. 4D, respectively.

According to FIG. 4A, FIG. 4B, FIG. 4C and FIG. 4D, it is known that the sizes of each kind of the films in different aqueous liquids changes significantly with increasing time.

Based on the diameters of the films before the time point of 7 days, it is known that the continuous swelling effects of the films was more significant in the aqueous solutions containing amphoteric-ions (zwitterion), such as Tri-HCl buffer, DMEM solution and DMEM solution containing 10% fetal bovine serum (cell culture medium). It is assumed that the aqueous solutions containing amphiphilic ions have more significant hydrogen bonding effects among collagen proteins, which leads to better hydration and faster swelling rates for the films.

Moreover, the hydration effect of the film is particularly significant in DMEM solution and DMEM solution containing 10% fetal bovine serum (cell culture medium). Since DMEM solution and cell culture medium contain amino acid components in addition to amphoteric ions, which also increase the hydration of collagen films, the swelling rates for the films therein much greater than those in other aqueous solutions (RO pure water, saline, Tri-HCl buffer or phosphate buffer solution).

Furthermore, the film diameters of the non-fibrous film prepared by Preparation example 1 (100 wt% collagen), the non-fibrous film prepared by Preparation example 2 (90 wt% collagen and 10 wt% polycarprolactone), the non-fibrous film prepared by Preparation example 7 (70 wt% collagen and 30 wt% polycarprolactone) prepared in Preparation example 7, and the non-fibrous film prepared by Preparation example 8 (non-fibrous film: 50 wt% collagen and 50 wt% polycarprolactone) in DMEM solution containing 10% fetal bovine serum determined at different time points shown in FIG. 4A, FIG. 4B, FIG. 4C and FIG. 4D are integrated in FIG. 5A.

Moreover, the swelling rate of each film was calculated based on the diameter of each film in DMEM solution containing 10% fetal bovine serum determined at the time point 60 minutes and at the time point 14 days. The results are shown in FIG. 5B.

In addition, compared to at the time point 60 minutes, the swelling proportions of each film in each kind of aqueous solutions at the time point 7 days and the time point 14 days are calculated, respectively. The formula for calculating the swelling proportion is shown below.

Swelling proportion (%) = (diameter of film at the second time point - diameter of film at the first time point)/diameter of film at the first time point*100

In this experiment, the first time point is 60 minutes and the second time point is 7 days or 14 days.

The results are shown in Table 4.

Table 4: Compared to at the time point 60 minutes, the swelling proportions of each film in each kind of aqueous solutions at the time point 7 days and the time point 14 days

| | | Swelling proportion (%) | | | | | |
|---|---|---|---|---|---|---|---|
| The film prepared in Preparation example 1 (100 wt% collagen) | Time | RO pure water | 0.9% physiological saline | Tri-HCl buffer solution | Phosphate buffer | DMEM | 10% fetal bovine serum DMEM solution containing |
| | 7 days | 3.16 | 3.00 | 12.39 | 3.46 | 13.99 | 13.86 |
| | 14 | 12.32 | 12.69 | 25.61 | 14.94 | 25.18 | 25.85 |

| | days | | | | | | |
|---|---|---|---|---|---|---|---|
| The film prepared in Preparation example 2 (90 wt% collagen and 10 wt% polycarprolactone) | Time | RO pure water | 0.9% physiological saline | Tri-HCl buffer solution | Phosphate buffer | DMEM | Tri-HCl buffer solution |
| | 7 days | 11.01 | 6.17 | 15.03 | 7.65 | 20.78 | 16.18 |
| | 14 days | 18.52 | 16.48 | 23.50 | 22.29 | 40.03 | 31.82 |
| The film prepared in Preparation example 3 (80 wt% collagen and 20 wt% polycarprolactone) | Time | RO pure water | 0.9% physiological saline | Tri-HCl buffer solution | Phosphate buffer | DMEM | DMEM solution containing 10% fetal bovine serum |
| | 7 days | 4.20 | 4.37 | 6.63 | 5.83 | 9.86 | 9.04 |
| | 14 days | 11.34 | 14.26 | 21.67 | 17.62 | 28.02 | 23.42 |

| The film prepared in Preparation example 7 (70 wt% collagen and 30 wt% polycarprolactone) | Time | RO pure water | 0.9% physiological saline | Tri-HCl buffer solution | Phosphate buffer | DMEM | Tri-HCl buffer solution |
|---|---|---|---|---|---|---|---|
| | 7 days | 0.10 | 7.04 | 7.38 | 3.35 | 12.19 | 15.00 |
| | 14 days | 11.70 | 16.91 | 16.85 | 20.58 | 39.73 | 41.64 |
| Preparation example 8 (50 wt% collagen and 50 wt% polycarprolactone) | Time | RO pure water | 0.9% physiological saline | Tri-HCl buffer solution | Phosphate buffer | DMEM | Tri-HCl buffer solution |
| | 7 days | -4.48 | -3.53 | -2.13 | -3.47 | 3.89 | 4.86 |
| | 14 days | 4.60 | 4.64 | 4.65 | 3.97 | 11.29 | 12.34 |

FIG. 5A, FIG. 5B and Table 4 show that the swelling proportions of the films containing 70-90 wt% collagen are greater than that of films containing 100 wt% collagen, and the swelling proportions of the films in each kind of aqueous liquids also increase with the increase in the content of the polyester polymer.

Accordingly, it is known that that the addition of the polyester polymer can impede the crosslinking among collagens and thus affect the intermolecular forces of collagen, resulting in an increase in the swelling rate of the film containing polyester polymers after absorption of the liquid.

In contrast, the collagen film containing 100 wt% is more fully cross-linked. However, because the hexafluoroisopropanol used as the solvent in the film preparation can make the film contain both natural collagen and denatured collagen (refer to FIG. 1A and FIG. 1B), 100 wt% collagen film immersed in a liquid still has swelling properties, but its swelling proportion is lower than those of the films containing 70-90 wt% collagen.

With regard to the film containing 50 wt% collagen, since the polyester polymer content of the film accounts for half of the total film, it has a higher degree of influence on the film properties. The polyester polymer is a hydrophobic material and does not absorb water and swell, and thus the film containing 50 wt% collagen only shows a slight increase in size after 7 days (168 hours) of immersion in the liquid.

(2) With regard to the films prepared in Preparation examples 4, 5 and 6

According to the diameters of the non-fibrous film prepared by Preparation example 4 (80 wt% collagen and 20 wt% polycarprolactone, crosslinking for 1.5 hours), the non-fibrous film prepared by Preparation example 5 (80 wt% collagen and 20 wt% polycarprolactone, crosslinking for 2 hours) and the non-fibrous film prepared by Preparation example 6 (80 wt% collagen and 20 wt% polycarprolactone, crosslinking for 3 hours) in DMEM solution containing 10% fetal bovine serums determined at the time point 14 days and the time point 60 minutes, the swelling rates and swelling proportion per unit time of the respective films were calculated.

Swelling proportion per unit time is calculated based on the formula shown below.

Swelling proportion per unit time (%hour) = swelling rate/film diameter at the time point 60 minutes * 100

Film diameter at the time point 60 minutes: initial diameter of the film after immersion of the film in an aqueous liquid
The results are shown in FIG. 6

FIG. 6 shows that the swelling rates of the films subjected to different cross-linking treatments with different time are different, and the swelling rate is higher for films with shorter cross-linking time. Therefore, it is known that the swelling rate of the film can be adjusted by the time of crosslinking for the film.

(3) With regard to the films prepared in Preparation examples 9 and 10

The film diameters of the non-fibrous film prepared in Preparation example 9 (90 wt% collagen and 10 wt% polylactic acid) determined in PBS buffer at different time points are as shown in FIG. 7.

Compared to at the time point 1 hour, the swelling proportions of the non-fibrous film prepared in Preparation example 9 (90 wt% collagen and 10 wt% polylactic acid) and the non-fibrous film prepared in Preparation example 10 (70 wt% collagen and 30 wt% polylactic acid) in PBS buffer at the time point 7 days and the time point 14 days are calculated, respectively. The results are shown in Table 5.

Table 5: Compared to at the time point 1 hour, the swelling proportions of the non-fibrous film prepared in Preparation example 9 (90 wt% collagen and 10 wt% polylactic acid) and the non-fibrous film prepared in Preparation example 10 (70 wt% collagen and 30 wt% polylactic acid) in PBS buffer at the time point 7 days and the time point 14 days.

|  |  | Swelling proportions (%) |
| --- | --- | --- |
| The film prepared in Preparation example 9 (90 wt% collagen and 10 wt% polylactic acid) | Time | PBS |
|  | 7 days | 10.41 |
|  | 14 days | 11.37 |
| The film prepared in Preparation example 10 (70 wt% collagen and 30 wt% polylactic acid) | Time | PBS |
|  | 7 days | 0.75 |
|  | 14 days | -0.87 |

According to FIG. 7 and Table 5, it is known that the film sizes of the collagen films containing polylactic acid in the aqueous liquid also increase with time.

(4) With regard to the films prepared in Comparative preparation example 3 and 4

The areas of the electrospun films (100 wt% collagen) prepared in Comparative preparation example 3 and the electrospun films (50 wt% collagen and 50 wt% polycarprolactone) prepared in Comparative preparation example 4 determined in PBS buffer at different time points are shown in FIG. 8.

Based on FIG. 8, it is known that the electrospun films have no swelling effect.

Example 5

Cell Culture Test

The films prepared in the Preparation example and the Comparative preparation example were subjected to cell culture tests, respectively.

1. Methods

Fibroblasts (HSF p7) were cultured in DMEM high glucose medium containing 10% fetal bovine serum and penicillin-streptomycin (100 U/mL).

The film to be tested was immersed in 75% ethanol for 15 minutes, and then the ethanol was removed and allowed to evaporate from the film to dry. When the drying step was completed, the sterilization of the film to be tested is completed.

Next, the films were immersed in sterile water for 40 minutes and then immersed in cell culture medium for 15 minutes. After that, the films were placed into the holes of a 12-hole plate and fixed with plastic rings. The fibroblasts (HSF p7) were inoculated into the holes with the films of the 12-hole plate mentioned above, at an inoculation density of 50,000 cells/cm$^2$. The fibroblasts (HSF p7) were inoculated at the same inoculation density in the holes without film of the 12-well plate mentioned above as a control group. After the on the next day, after the cells attached, the plastic rings for fixation were removed.

Changes of cells on the films were observed through PKH67 live cell staining. The cell morphology was photographed with a fluorescent microscope on Day 1, 3, 7 and 14 and the changes in material sizes were determined.

2. Results

(1) With regard to the films prepared in Preparation example 3

The results of the cell culture test of the non-fibrous film prepared in prepared in Preparation example 3 are shown in FIG. 9A and FIG. 9B.

FIG. 9A shows photographs of the growth status of cells on the non-fibrous film prepared in prepared in Preparation example 3 (80 wt% collagen and 20 wt% polycarprolactone) and cells on the culture plate on Day 1, 3, 7 and 14 in the cell culture test.

FIG. 9B shows photographs of the growth status of cells on the non-fibrous film prepared in prepared in Preparation example 3 (80 wt% collagen and 20 wt% polycarprolactone) and on the culture plate on Day 3 in the cell culture test.

According to FIG. 9A and FIG. 9B, it is known that the cells on the film of the present disclosure grow and arrange in the same direction. In the cell culture medium, the size of the film continuously increase over time, and the increase in the size of the film causes a physical mechanical stress on the cells to allow the cells show a property of growing in the same direction. In contrast, the growth status of cells on the culture plate is more disorganized.

(2) With regard to the film prepared in Comparative preparation example 2

The result of the cell culture test of the non-fibrous film prepared in Comparative preparation example 2 (90 wt% collagen and 10 wt% polycarprolactone (acidic aqueous solution as a solvent)) is shown in FIG. 10.

According to FIG. 10, it is known that similar to the growth status of cells on culture plate, the growth status of cells on the non-fibrous film prepared in Comparative preparation example 2 (90 wt% collagen and 10 wt% polycarprolactone (acidic aqueous solution as a solvent)) is more disorganized and only occasionally arranged in a directional manner.

Based on the foregoing, it is known that, during cell culture, since the film of the present disclosure have changes in size, it provides the cells grow thereon with a physical mechanical stress and a stimulus of structural changes on the surface to which the cells are attached to allow the cells grow in the same direction and to promote cell differentiation.

It will be apparent to those skilled in the art that various modifications and variations can be made to the disclosed embodiments. It is intended that the specification and examples be considered as exemplary only, with a true scope of the disclosure being indicated by the following claims.

**Claims**

1. A non-fibrous film, which is prepared by a method,

   wherein the method comprises:

      (a) preparing a mixture solution,
      a solute of which comprises:

         a collagen and a polyester polymer,
         wherein a content of the polyester polymer in the non-fibrous film is 1-60 wt%, and
         a solvent of which comprises a perfluorocarbon solvent; and

      (b) drying the mixture solution in a fume hood to form a film,

   wherein the film has a swelling rate of 1-200 $\mu$m/hour or a swelling proportion per unit time of 0.1-2%/hour in an aqueous liquid.

2. The non-fibrous film as claimed in claim 1, wherein a concentration of the solute in the mixture solution is 0.5-20 wt%.

3. The non-fibrous film as claimed in claim 1 or 2, wherein the collagen comprises at least one of the following: type I collagen, type II collagen and type III collagen.

4. The non-fibrous film as claimed in any one of claims 1-3, wherein the composition of the non-fibrous film consists of the collagen and the polyester polymer, and a weight ratio of the collagen to the polyester polymer is 95:5, 90:10, 80:20, 70:30 or 50:50.

5. The non-fibrous film as claimed in any one of claims 1-4, wherein the polyester polymer comprises at least one of the following:
   polycarprolactone, polylactic acid, polyglycolic acid, polyhydroxybutyrate and polyhydroxyvalerate.

6. The non-fibrous film as claimed in any one of claims 1-5, wherein the non-fibrous film is a porous film and the polyester polymer is polycarprolactone, wherein a molecular weight of the polycarprolactone is 50,000-200,000 Da.

7. The non-fibrous film as claimed in any one of claims 1-5, wherein the non-fibrous film is a non-porous film and the polyester polymer is polylactic acid, wherein an intrinsic viscosity of the polylactic acid is 0.15-7.0 dl/g.

8. The non-fibrous film as claimed in any one of claims 1-7, wherein the perfluorocarbon solvent comprises hexafluoroisopropanol, hexafluoroacetone, 1,1,1-trichlorotrifluoroacetone, trifluoroacetic acid, perfluoroperhydrophenanthrene or perfluoro(methylcyclohexane).

9. The non-fibrous film as claimed in any one of claims 1-8, wherein the aqueous liquid comprises water, buffer or medium.

10. The non-fibrous film as claimed in any one of claim 1-9, wherein the method further comprises after the step (b), performing a crosslinking treatment on the film, wherein the time for the crosslinking treatment is 0.1-10 hours.

11. A cell sheet, comprising:

    the non-fibrous film as claimed in any one of claims 1-10; and
    at least one cell layer on the non-fibrous film,
    wherein the cell layer is composed of a plurality of cells, and the plurality of cells are arranged in the same direction.

12. The cell sheet as claimed in claim 11, wherein the cells comprise fibroblast, myoblasts, epithelial cells, endothelial cells, progenitor cells, tenocyte, stem cells, mesenchymal stem cells, bone marrow stem cells or adipose derived stem cells.

**Patentansprüche**

1. Nichtfaseriger Film, der durch ein Verfahren hergestellt wird, wobei das Verfahren die Schritte aufweist:

   (a) Herstellen einer Mischlösung,

   von der ein gelöster Stoff ein Kollagen und ein Polyesterpolymer aufweist,
   wobei ein Gehalt des Polyesterpolymers in dem nichtfaserigen Film 1 bis 60 Gew.-% beträgt; und
   von der ein Lösungsmittel ein Perfluorkohlenstoff-Lösungsmittel aufweist; und

   (b) Trocknen der Mischlösung in einem Abzug, um einen Film auszubilden, wobei der Film eine Quellrate von 1 bis 200 $\mu$m/Stunde oder eine Quellquote pro Zeiteinheit von 0,1 bis 2%/Stunde in einer wässrigen Flüssigkeit aufweist.

2. Nichtfaseriger Film nach Anspruch 1, wobei die Konzentration des gelösten Stoffs in der Mischlösung 0,5 bis 20 Gew.-% beträgt.

3. Nichtfaseriger Film nach Anspruch 1 oder 2, wobei das Kollagen mindestens eines der folgenden aufweist: Kollagen Typ-I, Kollagen Typ-II und Kollagen Typ-III.

4. Nichtfaseriger Film nach einem der Ansprüche 1 bis 3, wobei die Zusammensetzung des nichtfaserigen Films aus dem Kollagen und dem Polyesterpolymer besteht und das Gewichtsverhältnis des Kollagens zum Polyesterpolymer 95:5, 90:10, 80:20, 70:30 oder 50:50 beträgt.

5. Nichtfaseriger Film nach einem der Ansprüche 1 bis 4, wobei das Polyesterpolymer mindestens eine der folgenden Komponenten aufweist: Polycarprolacton, Polymilchsäure, Polyglykolsäure, Polyhydroxybutyrat und Polyhydroxyvalerat.

6. Nichtfaseriger Film nach einem der Ansprüche 1 bis 5, wobei der nichtfaserige Film ein poröser Film ist und das Polyesterpolymer Polycarprolacton ist, wobei das Molekulargewicht des Polycarprolactons 50000 bis 200000 Da beträgt.

7. Nichtfaseriger Film nach einem der Ansprüche 1 bis 5, wobei der nichtfaserige Film ein nicht-poröser Film ist und das Polyesterpolymer Polymilchsäure ist, wobei die intrinsische Viskosität der Polymilchsäure 0,15 bis 7,0 dl/g beträgt.

8. Nichtfaseriger Film nach einem der Ansprüche 1 bis 7, wobei das Perfluorkohlenstoff-Lösungsmittel Hexafluorisopropanol, Hexafluoraceton, 1,1,1-Trichlortrifluoraceton, Trifluoressigsäure, Perfluorperhydrophenanthren oder Perfluor(methylcyclohexan) aufweist.

9. Nichtfaseriger Film nach einem der Ansprüche 1 bis 8, wobei die wässrige Flüssigkeit Wasser, einen Puffer oder ein Medium aufweist.

10. Nichtfaseriger Film nach einem der Ansprüche 1 bis 9, wobei das Verfahren ferner nach dem Schritt (b) das Ausführen einer Vernetzungsbehandlung bezüglich des Films aufweist, wobei die Zeit für die Vernetzungsbehandlung 0,1 bis 10 Stunden beträgt.

11. Zellfolie, mit:

    dem nichtfaserigen Film nach einem der Ansprüche 1 bis 10; und
    mindestens einer Zellschicht auf dem nichtfaserigen Film,
    wobei die Zellschicht aus einer Vielzahl von Zellen zusammengesetzt ist und die Vielzahl von Zellen in der gleichen Richtung angeordnet sind.

**12.** Zellfolie nach Anspruch 11, wobei die Zellen Fibroblasten, Myoblasten, Epithelzellen, Endothelzellen, Progenitorzellen, Tenozyten, Stammzellen, mesenchymale Stammzellen, Stammzellen aus dem Knochenmark oder aus Fettgewebe gewonnene Stammzellen aufweisen.

**Revendications**

**1.** Film non fibreux, qui est préparé par un procédé,

dans lequel le procédé comprend :

(a) la préparation d'une solution de mélange,
dont un soluté comprend :

un collagène et un polymère de polyester,
dans lequel une teneur du polymère de polyester en le film non fibreux est de 1 à 60 % en masse, et
dont un solvant comprend un solvant perfluorocarboné ; et

(b) le séchage de la solution de mélange dans une hotte d'aspiration pour former un film,

dans lequel le film a une vitesse de gonflement de 1 à 200 $\mu$m/heure ou une proportion de gonflement par unité de temps de 0,1 à 2 %/heure dans un liquide aqueux.

**2.** Film non fibreux selon la revendication 1, dans lequel une concentration du soluté dans la solution de mélange est de 0,5 à 20 % en masse.

**3.** Film non fibreux selon la revendication 1 ou 2, dans lequel le collagène comprend au moins l'un parmi les suivants : collagène de type I, collagène de type II et collagène de type III.

**4.** Film non fibreux selon l'une quelconque des revendications 1 à 3, dans lequel la composition du film non fibreux est constituée du collagène et du polymère de polyester, et un rapport massique du collagène au polymère de polyester est de 95:5, 90:10, 80:20, 70:30 ou 50:50.

**5.** Film non fibreux selon l'une quelconque des revendications 1 à 4, dans lequel le polymère de polyester comprend au moins l'un parmi les suivants : polycarprolactone, acide polylactique, acide polyglycolique, polyhydroxybutyrate et polyhydroxyvalérate.

**6.** Film non fibreux selon l'une quelconque des revendications 1 à 5, dans lequel le film non fibreux est un film poreux et le polymère de polyester est la polycarprolactone, dans lequel une masse moléculaire de la polycarprolactone est de 50 000 à 200 000 Da.

**7.** Film non fibreux selon l'une quelconque des revendications 1 à 5, dans lequel le film non fibreux est un film non poreux et le polymère de polyester est l'acide polylactique, dans lequel une viscosité intrinsèque de l'acide polylactique est de 0,15 à 7,0 dl/g.

**8.** Film non fibreux selon l'une quelconque des revendications 1 à 7, dans lequel le solvant perfluorocarboné comprend l'hexafluoroisopropanol, l'hexafluoroacétone, la 1,1,1-trichlorotrifluoroacétone, l'acide trifluoroacétique, le perfluoroperhydrophénanthrène ou le perfluoro(méthylcyclohexane).

**9.** Film non fibreux selon l'une quelconque des revendications 1 à 8, dans lequel le liquide aqueux comprend de l'eau, un tampon ou un milieu.

**10.** Film non fibreux selon l'une quelconque des revendications 1 à 9, dans lequel le procédé comprend en outre après l'étape (b) la réalisation d'un traitement de réticulation sur le film, dans lequel la durée du traitement de réticulation est de 0,1 à 10 heures.

**11.** Feuille cellulaire, comprenant :

le film non fibreux selon l'une quelconque des revendications 1 à 10 ; et
au moins une couche cellulaire sur le film non fibreux,
dans lequel la couche cellulaire est composée d'une pluralité de cellules, et la pluralité de cellules sont agencées dans la même direction.

12. Feuille cellulaire selon la revendication 11, dans lequel les cellules comprennent les fibroblastes, les myoblastes, les cellules épithéliales, les cellules endothéliales, les cellules progénitrices, les ténocytes, les cellules souches, les cellules souches mésenchymateuses, les cellules souches de moelle osseuse ou les cellules souches dérivées de tissu adipeux.

FIG. 1A

FIG. 1B

FIG. 2

FIG. 3

FIG. 4A

EP 4 023 266 B1

FIG. 4B

FIG. 4C

FIG. 4D

FIG. 5A

FIG. 5B

FIG. 6

FIG. 7

EP 4 023 266 B1

FIG. 8

EP 4 023 266 B1

Cultured on a plate

| 1 days | 3 days | 7 days | 14 days |

100X

Cultured on a film with a collagen content of 80 wt%

100X

FIG. 9A

EP 4 023 266 B1

3 days，100X

Cultured on a plate

Cultured on a film with a
collagen content of 80 wt%

FIG. 9B

6 days，100X

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 107325307 A

### Non-patent literature cited in the description

- **OLENA S. RABOTYAGOVA et al.** Collagen structural hierarchy and susceptibility to degradation. susceptibility to degradation by ultraviolet radiation. *Materials Science and Engineering C,* 2008, vol. 28, 1420-1429